# EUROPEAN PATENT APPLICATION

(11) **EP 3 888 619 A1**
(43) Date of publication of application: **06.10.2021**
(21) Application number: 20167826.5
(22) Date of filing: 02.04.2020
(51) Int. Cl.: A61K 8/02, A61K 8/19, A61K 8/24, A61K 8/25, A61K 8/85, A61Q 11/00

(54) **ORAL CARE DEVICE**

(71) Applicant: Unilever Global IP Ltd, Wirral, CH62 4ZD (GB)
(72) Inventor: GROVES, Brian Joseph, Wirral, Merseyside CH63 3JW (GB); LIMER, Adam John, Wirral, Merseyside CH63 3JW (GB)
(74) Representative: Tansley, Sally Elizabeth

(57) **Abstract**

A system for whitening the teeth the system comprising a delivery device and a separate system composition; the delivery device comprising:
i) a strip of an orally acceptable flexible material, having a strip surface capable of being applied to a tooth surface; and
ii) a phosphate source;
the activator system comprising:
iii) less than 1wt% of the total activator system of water; and
iv) a phosphate source

## Description

### Field of the Invention

The invention relates to an oral care products for the remineralisation and whitening of teeth.

### Background of the Invention

Teeth are important both functionally and aesthetically. There is a need for strong healthy teeth that appear white and glossy.

WO2016/192923 discloses a delivery device for delivering a enamel regeneration system to the surfaces of teeth. The device is a strip having the enamel regeneration system deposited upon the strip surface thereof, and/or impregnated into its structure. Similarly whitening and enamel regeneration systems comprising strips are disclosed in WO2016/192924 and WO2016/19295.

However there remains the need for a simple and effective way to enable enamel regeneration of the teeth and to whiten the teeth.

### Summary of the Invention

Accordingly, the present invention relates to a system for whitening the teeth the system comprising a delivery device and a separate activator composition;
the delivery device comprising:
i) a strip of an orally acceptable flexible material, having a strip surface capable of being applied to a tooth surface; and
ii) a phosphate source;
   the activator system comprising:
iii) less than 1wt% of the activator system of water; and
iv) a phosphate source.

### Detailed Description of the Invention

The activator system comprises an anhydrous base formulation in that the activator base comprises less than 1wt% of the activator system of water; preferably less than 0.5 wt%, more preferably less than 0.1 wt% of the total activator system.

The activator system preferably comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 1 to 12%, and most preferably, from 2 to 9% by weight of the composition, based on total weight of the activator system in which it is included and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

It is preferable the activating system comprises a calcium source that is insoluble or slightly soluble in water.

Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. In a preferred embodiment the calcium source is calcium silicate. In a more preferred embodiment, the calcium silicate used is (CaSᵢC₃) whereby the same is made commercially available under the name Microcal ET by Ineos Silicas, Ltd.

In yet another preferred embodiment, the calcium source is insoluble calcium silicate, present as the composite material calcium oxide-silica (CaO-SiO₂) as described in commonly-owned application Publication No. 2008/015117.

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often-preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition is typically from 0.1 to 50%, and preferably, from 1 to 30%, and most preferably, from 5 to 20% by weight of the oral care composition based on total weight of the oral care composition and including all ranges subsumed therein.

A preferred form of calcium silicate is calcium silicate coated TiO₂. Examples of preferred forms of calcium silicate coated TO02 are disclosed in WO2012/031786 and WO20 12/031785.

The oral care composition described herein may comprise ingredients which are common in the art, such as:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc;
▪ biomolecules, e.g. bacteriocins, antibodies, enzymes, etc ;
▪ flavours, e.g., peppermint and spearmint oils;
▪ proteinaceous materials such as collagen;
▪ preservatives;
▪ opacifying agents;
▪ colouring agents like FD&C blue, yellow and/or red dyes/colorants;
▪ pH-adjusting agents;
▪ sweetening agents;
▪ surfactants, such as anionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
▪ other optional ingredients that may be included are, e.g., bleaching agents such as peroxy compound, e.g., potassium peroxydiphosphate, effervescing systems such as sodium bicarbonate/citric acid systems, colour change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care system of the present invention, in particular the activation system and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

The composition used in the layers of the invention are prepared by conventional methods of making oral care formulations. Such methods include mixing the ingredients under moderate shear and atmospheric pressure.

The delivery device of the invention comprises a strip of an orally acceptable flexible material. The surface of the strip is capable of being applied to a tooth surface.

The device comprises a phosphate source, preferably the phosphate resides in a layer as part of the device. The phosphate I of the device is as described for the activator system. preferably the same phosphate is used in the device and in the activator system. Typically, the phosphate source makes up from 0.5 to 15%, and preferably, from 1 to 12%, and most preferably, from 2 to 9% by weight of the composition, based on total weight of the composition of the layer in which it is included and including all ranges subsumed therein.

Preferably the device has an elongate shape of a length sufficient that when placed against the front surface of a user's teeth it extends across a plurality of teeth, and of sufficient width that it extends at least from the gumline of the teeth to the crowns of the teeth. The elongate shape is such that it minimises the need for subsequent applications and time to cover all the user's teeth.

In a further embodiment, the strip is such that it can be sufficient that when placed against the front surface of a user's teeth it extends across a plurality of teeth, and of sufficient width that it extends at least from the front gumline of the teeth to the crowns of the teeth and to the gumline behind the user's teeth leading to total coverage of the teeth above the gumline.

Preferably the application device is rectangular in shape.

Preferably the strip/device has the enamel regeneration system deposited upon the strip surface thereof as a layer or more preferably multiple layers.

In a preferred embodiment, the device is a strip comprising a plurality of layers.

Preferably the device comprises at least two layers; one layer comprises a non-dissolving backing film, a second layer comprises a component of the enamel regeneration system. More preferably the strip comprises third protective layer it is particularly preferred if the third protective layer comprises a water-soluble polymer.

It is preferable if the non-dissolving backing layer comprises materials such as polymers, natural and synthetic wovens, non-wovens, foil, paper, rubber, and combinations thereof. The strip of material may be a single layer of material or a laminate of more than one layer. Generally, the strip of material is substantially water impermeable. The material may be any type of polymer that is compatible with tooth whitening actives and is sufficiently flexible to be shaped and applied to the tooth surface. The material may comprise a single polymer or a mixtures of polymers. Suitable polymers include, but are not limited to, polyethylene, polypropylene, ethylvinylacetate, ethylvinyl alcohol, polyesters, polyamides, fluoroplastics and combinations thereof. Preferably, the material is polyethylene, in particular polyethylene terephthalate.

The strip of material is generally less than about 1 mm thick, preferably less than about 0.05 mm thick, and more preferably from about 0.001 to about 0.03 mm thick.

In one embodiment, the device comprises a third layer. The third layer preferably comprises a water-soluble polymer or polymers. Suitable water soluble polymers may be natural or synthetic. Suitable natural hydrocolloids and water soluble polymers include cellulosic material, a polysaccharide, a gum, a protein, a starch or a glucan. Examples include but are not limited to carboxymethyl cellulose, hydroxyl methyl cellulose, hydroxyethyl cellulose, hydroxypropyl cellulose, hydroxypropylmethyl cellulose, gum Arabic, xanthan gum, karaya gum, tragacanth, acacia, carrageenan, guar gum, locust bean gum, pectin, alginates, polydextrose, dextrin, dextran, amylose, chitin, chitosan, levan, elsinan, collagen, gelatin, zein, gluten, soy protein isolate, whey protein isolate, casein and pullan. Suitable water soluble synthetic polymers include but are not limited to poly(vinyl pyrrolidone), poly(vinyl alcohol), poly(acrylic acid), polyacrylates, Methylmethacrylate copolymer, carboxyvinyl polymer, polyethylene oxide and polyethylene glycol.

The advantage of having a water-soluble layer and a non-water soluble layer is that in the mouth the water-soluble layer dissolves in the saliva and the ingredients enclosed therein (the phosphate) can react with the ingredients in the second layer (silicate) to aid the in-situ regeneration of enamel on the teeth. Thus, the system provides an enamel regeneration system that can be used with the minimum of water, can be easily stored and is stable on storage.

Preferably the composition in one of the compositions within the layers of the invention comprises titanium dioxide. In particular, the layer comprising an adhesive such as polyethylene glycols or celluloses/hydroxyalkyl celluloses.

It is preferable if the thickness of the first layer is less than about 1 mm thick, preferably less than about 0.05 mm thick, and more preferably from about 0.001 to about 0.03 mm thick, second layer is less than about 1 mm thick, preferably less than about 0.5 mm thick, and more preferably from about 0.001 to about 0.3 mm thick and third layer (if present) is less than about 1 mm thick, preferably less than about 0.5 mm thick, and more preferably from about 0.001 to about 0.3 mm thick.

### Mode of Use

The invention provides a method of whitening and remineralising teeth. The activator system is applied to the strips of the invention. The activated strips as described above are applied to the surface of the teeth in sustained contact.

In the context of the present invention sustained contact means the product is left on the teeth for 1 to 60 minutes, preferably, about 5 to 45 minutes, more preferably 10 to 30 minutes before being removed.

Preferably the application of the oral care product of the invention is carried out once daily for a period of several consecutive days, in addition to a regular regime of tooth brushing (preferably at least twice daily).

Typically, use (for a period of about two weeks to one month) of the device with the oral care composition of the present invention will result in a new hydroxyapatite layer on teeth that is from 0.5 to 20 microns, and preferably, from 0.75 to 5 microns, including all ranges subsumed therein.

### Detailed Description of Non-limiting Embodiments

The invention will now be illustrated by the following non-limiting Examples:

### Examples

A simulated oral fluid was prepared by adding 1.9L of water to a glass beaker. The following materials were added one by one with continuous stirring, allowing sufficient time for each chemical to fully dissolve before adding the next. Sodium chloride 16.07g, sodium hydrogen carbonate 0.7g, potassium chloride 0.448g, potassium hydrogen phosphate 2.56g, magnesium chloride hexahydrate 0.622g, 1M hydrochloric acid 40ml, calcium chloride 0.1998g and sodium sulfate 0.1434g. The pH was adjusted to 7.0 using saturated TRIS buffer and the total volume made up to 2L using a volumetric flask.

Human extracted incisors and premolars, obtained for research purposes and obtained with informed consent in accordance with the Human Tissue Act were mounted in plastic cuvettes using the following method. The cuvettes were cut to approximately 15mm height. A commercial fixative (Simplex Rapid) was mixed 2 parts powder to 1 part liquid as described in the manufacturer's instructions. The cuvettes were then completely filled with the resultant solution and left for approximately 10 minutes to allow the fixative to partly set. At this time one tooth root was completely immersed into the fixative, ensuring that the labial side of the tooth was positioned close to the front of the cuvette. Complete setting of the fixative was achieved after 20 minutes. Any exposed dentine is then sealed with clear nail varnish. The teeth are stored in water to prevent dehydration.

A pair of formulations were prepared by combining the following:

| **Ingredient** | **Example 1A %w/w** | **Example 1B %w/w** |
|---|---|---|
| Glycerol | 32.745 | 31.245 |
| PEG 400 | 32.745 | 31.245 |
| Calcium Silicate | 11 | 11 |
| Calcium silicate coated TiO2 | 20 | 20 |
| Sodium monofluorophosphate | 1.11 | 1.11 |
| Calcium Phosphate | 2 | 2 |
| Sodium Saccharine | 0.1 | 0.1 |
| Flavor | 0.3 | 0.3 |
| Anhydrous Mono Sodium Phosphate | 0 | 1.50 |
| Anhydrous Tri Sodium Phosphate | 0 | 1.50 |
| Total | 100 | 100 |

The glycerol and PEG 400 were added to the Esco-Labor 1L mixing vessel and stirring at room temperature the powders are added slowly and mixed to remove any lumps.

### Example 1:

A strip was produced by preparing two formulations:

### Formulation 1:

| **Ingredient** | **%w/w** |
|---|---|
| Purified Water | 97.498% |
| Glycerine | 0.5% |
| Benzyl Alcohol EP/USP/NF | 0.001% |
| Phenoxyethanol | 0.001% |
| Hydroxyethyl Cellulose | 2.000% |
| Total | 100% |

Water was added to a mixing vessel followed by glycerine, Benzyl Alcohol,
Phenoxyethanol and mixed to disperse. Subsequently hydroxyethyl cellulose was slowly added to the vessel and mixed to disperse.

### Formulation 2:

| **Ingredient** | **Formulation 2A %w/w** | **Formulation 2B %w/w** |
|---|---|---|
| Purified Water | 57.75% | 61.75% |
| Glycerine | 16.000% | 16.000% |
| Hydroxyethyl Cellulose | 1.000% | 1.000% |
| Silica | 17.830% | 17.830% |
| Polyvinylpyrrolidone | 2.000% | 2.000% |
| Trisodium Phosphate | 2% | 0% |
| Monosodium Phosphate | 2% | 0% |
| Aroma | 0.250% | 0.250% |
| Benzyl Alcohol | 0.300% | 0.300% |
| Phenoxyethanol | 0.300% | 0.300% |
| Ethylhexylglycerin | 0.300% | 0.300% |
| Sodium Fluoride | 0.100% | 0.100% |
| PEG-40 Hydrogenated Castor Oil | 0.100% | 0.100% |
| Sodium Saccharin | 0.100% | 0.100% |
| Total | 100% | 100% |

Water was added to a mixing vessel followed by PEG-40 Hydrogenated Castor Oil, glycerine, ethylhexyl glycerine, phenoxyethanol, Benzyl Alcohol, flavour, monosodium phosphate, trisodium phosphate, sodium fluoride, sodium saccharine, silica and mixed to disperse. Subsequently hydroxyethyl cellulose and polyvinylpyrrolidone was slowly added to the vessel and mixed to disperse.

A film of formulation 1 was applied to a PET film. On this film was placed a layer of non-woven Rayon fabric and a film of formulation 2 applied. The sample was dried in an oven to a water content of 28-30wt%. The resulting structure has four layers, a PET film, a layer of formulation 1, a layer or rayon and a layer of formulation 2. The resulting structure can be pealed from the PET layer to give a three-layer structure.

The product was tested as follows:
A 3:1 water:tooth-paste slurry was prepared using a Signal toothpaste marketed as Signal Anti-Caries. Each tooth was brushed by hand using a toothbrush for 5 seconds and soaked in slurry for 115 seconds. After the teeth were rinsed in 40 ml water and mixed gently for 1 minute. Two pieces of the four-layer structure were cut to a 10 x 3.5cm piece. After brushing the teeth with toothpaste and immediately before use, 0.5g of Example B was applied to the exposed silica layer of each fabric sample using a dropping pipette and spread evenly with a spatula. The sample was pealed from the PET carrier and wrapped around 5 human teeth for 15 minutes at 37°C. After the samples were removed from the fabric, rinsed in 40 ml water and mixed gently for 1 minute. Samples were then incubated in simulated oral fluid for 5 hours. The application process was repeated 5 times in total. Colour was measured via chromameter at baseline and after slurry application and incubation. L*a*b* colour parameters were converted to WIO whiteness indices to allow comparison between samples. Colour change is expressed as ΔWIO = WIO(slurry application)-WIO(baseline).

| | **1 Application** | **3 Applications** |
|---|---|---|
| Formulation 2A | 5.38 ± 1.11 | 7.17 ± 0.76 |
| Formulation 2B | 3.25 ± 1.06 | 3.56 ± 0.97 |

The examples demonstrate that Formulation 2A which contains sodium phosphate gives a greater increase in whiteness than Formulation 2B.

### Example 2:

A strip was produced by preparing two formulations:

### Formulation 3:

| **Ingredient** | **%w/w** |
|---|---|
| Purified Water | 82.248% |
| Titanium Dioxide | 0.500% |
| Glycerine | 2.000% |
| PEG-1500 NF (Polyethylene Glycol 1500) | 12.000% |
| White Beeswak, NF | 0.500% |
| PEG-40 Hydrogenated Castor Oil | 0.250% |
| Benzyl Alcohol EP/USP/NF | 0.001% |
| Phenoxyethanol | 0.001% |
| Hydroxyethyl Cellulose | 2.500% |
| Total | 100% |

Water was added to a mixing vessel followed by PEG-40 Hydrogenated Castor Oil, glycerine, PEG, beeswax, Benzyl Alcohol, Phenoxyethanol, titanium dioxide and mixed to disperse. Subsequently hydroxyethyl cellulose was slowly added to the vessel and mixed to disperse.

### Formulation 4:

| **Ingredient** | **%w/w** |
|---|---|
| Purified Water | 58.100% |
| Glycerine | 16.000% |
| Hydroxyethyl Cellulose | 1.000% |
| Silica | 17.830% |
| Polyvinylpyrrolidone | 2.000% |
| Trisodium Phosphate | 1.750% |
| Monosodium Phosphate | 1.750% |
| Aroma | 0.250% |
| Benzyl Alcohol | 0.300% |
| Phenoxyethanol | 0.300% |
| Ethylhexylglycerin | 0.300% |
| Sodium Fluoride | 0.220% |
| PEG-40 Hydrogenated Castor Oil | 0.100% |
| Sodium Saccharin | 0.100% |
| Total | 100% |

Water was added to a mixing vessel followed by PEG-40 Hydrogenated Castor Oil, glycerine, ethylhexyl glycerine, phenoxyethanol, Benzyl Alcohol, flavour, monosodium phosphate, trisodium phosphate, sodium fluoride, sodium saccharine, silica and mixed to disperse. Subsequently hydroxyethyl cellulose and polyvinylpyrrolidone was slowly added to the vessel and mixed to disperse.

A film of formulation 3 was applied to a PET film and dried in an oven to a water content of 28-30wt%. On this film was placed a layer of non-woven Rayon fabric and a film of formulation 4 applied. Again, the sample was dried in an oven to a water content of 28-30wt%. The resulting structure has four layers, a PET film, a layer of formulation 3, a layer or rayon and a layer of formulation 4. The resulting structure can be pealed from the PET layer to give a three-layer structure.

The product was tested as follows:
A 3:1 water:tooth-paste slurry was prepared using a Signal toothpaste marketed as Signal Anti-Caries. Each tooth was brushed by hand using a toothbrush for 5 seconds and soaked in slurry for 115 seconds. After the teeth were rinsed in 40 ml water and mixed gently for 1 minute. Two pieces of the four-layer structure were cut to a 10 x 3.5cm piece. After brushing the teeth with toothpaste and immediately before use, 0.5g of Example A or Example B was applied to the exposed silica layer of each fabric sample using a dropping pipette and spread evenly with a spatula. The sample was pealed from the PET carrier and wrapped around 5 human teeth for 15 minutes at 37°C. After the samples were removed from the fabric, rinsed in 40 ml water and mixed gently for 1 minute. Samples were then incubated in simulated oral fluid for 5 hours. The application process was repeated 5 times in total. Colour was measured via chromameter at baseline and after slurry application and incubation. L*a*b* colour parameters were converted to WIO whiteness indices to allow comparison between samples. Colour change is expressed as ΔWIO = WIO(slurry application)-WIO(baseline).

| | **1 Application** | **5 Applications** |
|---|---|---|
| Example A | 5.36 ± 0.59 | 8.35 ± 2.09 |
| Example B | 9.38 ± 1.18 | 18.66 ± 3.43 |

The examples demonstrate that Example B which contains sodium phosphate gives a greater increase in whiteness than Example A.

## Claims

1. A system for whitening the teeth the system comprising a delivery device and a separate system composition; the delivery device comprising:
i) a strip of an orally acceptable flexible material, having a strip surface capable of being applied to a tooth surface; and
ii) a phosphate source;
the activator system comprising:
iii) less than 1wt% of the total activator system of water; and
iv) a phosphate source

2. A system according to claim 1, the device comprising a plurality of layers.

3. A system according to claim 2, in which one layer of the device comprises a non-dissolving backing film.

4. A system according to claim 3 in which the non-dissolving backing film comprises polyethylene terephthalate.

5. A system according to any preceding claim in which the device comprises a layer comprising an adhesive polymer.

6. A system according to any preceding claim in which the phosphate source comprises trisodium phosphate, sodium dihydrogen phosphate or mixtures thereof.

7. A system according to any preceding claim in which the activator system further comprises a water insoluble and/or slightly soluble calcium source.

8. A system according to claim 7 in which the calcium source of the enamel regeneration system is calcium silicate.

9. A system according to any preceding claim in which the system further comprises a tooth whitening substance.

10. A system according to claim 9 in which the tooth whitening substance is calcium silicate coated TiO2.

11. A system according to claim 9 or claim 10 in which the tooth whitening substance is present in the activator system.

12. A system according to any preceding claim in which the device has an elongate shape of a length sufficient that when placed against the front surface of a user's teeth it extends across a plurality of teeth, and of sufficient width that it extends at least from the gumline of the teeth to the crowns of the teeth.

13. A system according to any preceding claim in which the device is substantially rectangular.
